# EUROPÄISCHE PATENTSCHRIFT

(11) **EP 1 024 743 B1**
(45) Veröffentlichungstag und Bekanntmachung des Hinweises auf die Patenterteilung: **14.01.2004**
(21) Anmeldenummer: 98954407.7
(22) Anmeldetag: 13.10.1998
(51) Int. Cl.: A61B 5/05

(54) **VORRICHTUNG ZUR FESTSTELLUNG EINES PERIKARDERGUSSES**
DEVICE FOR DETECTING PERICARDIAL EFFUSION
DISPOSITIF DE DETECTION D'UN EPANCHEMENT PERICARDIQUE

(30) Priorität: 24.10.1997 DE 19747172
(43) Veröffentlichungstag der Anmeldung: 09.08.2000
(73) Patentinhaber: Pulsion Medical Systems AG, 81829 München (DE)
(72) Erfinder: GÖDJE, Oliver, D-81377 München (DE)
(74) Vertreter: Kehl, Günther, Dipl.-Phys.
(86) Internationale Anmeldenummer: PCT/EP1998/006483
(87) Internationale Veröffentlichungsnummer: WO 1999/021475

(56) Entgegenhaltungen:
- EP-A- 0 487 429
- GB-A- 2 213 381
- US-A- 3 971 365
- US-A- 4 862 361
- US-A- 5 454 377

## Beschreibung

Die Erfindung betrifft eine Vorrichtung zur Feststellung eines Perikardergusses nach herzchirurgischen Eingriffen.

Für nahezu jeden herzchirurgischen Eingriff, Herzklappenersatz, Bypass-Chirugie, Transplantation oder Implantation einer Gefäßprothese ist die Eröffnung des Perikards notwendig. Zuvor müssen die Haut, das Unterhaut-Fettgewebe und das Brustbein durchtrennt werden. Nach Eröffnung des Perikards beginnt der eigentliche Eingriff. Zunächst erfolgt für alle Operationen in ähnlicher Weise der Anschluß der Herz-Lungen-Maschine. Hierzu werden die Aorta, in den rechten Vorhof oder die obere und untere Hohlvene und in die rechte Lungenvene jeweils großlumige Katheter eingebracht, über die im Anschluß das Blut zirkuliert. Nach Kühlen des Patienten auf eine Temperatur zwischen 18°C und 32°C wird dann die Operation vorgenommen. Hierzu werden bei einem Bypass-Eingriff die Herzkranzgefäße präpariert und mit den Bypässen verbunden, für den Herzklappenersatz wird die Aorta bzw. der rechte oder linke Vorhof eröffnet, bei der Transplantation werden alle größeren Gefäße, die von oder zum Herz führen, durchtrennt und mit dem neuen Herz verbunden. Nach durchgeführter Operation wird der Patient an der Herz-Lungen-Maschine wieder aufgewärmt und bei ausreichend stabilen Herz-Kreislauf-Verhältnissen von dieser wieder getrennt. Nach Einlegen mehrerer Drainagen zum Ableiten der Nachblutungen wird das Brustbein mit Drähten und die Haut mit Fäden verschlossen.

Wie aus dem geschilderten Ablauf eines herzchirurgischen Eingriffs ersichtlich ist, entstehen hierbei sehr große Wundflächen. Ist der Patient bereits am Herzen voroperiert, entstehen durch die Durchtrennen von bestehenden Verwachsungen noch größere Wundflächen.

Zu diesen Wundflächen, aus denen Blutungen möglich sind, kommt noch das Problem der Antikoagulation hinzu. Damit das Blut nicht in der Herz-Lungen-Maschine oder den Schläuchen gerinnt, wird das Blut des Patienten vorher medikamentös ungerinnbar gemacht. Nach Ende des Eingriffs wird diese Ungerinnbarkeit medikamentös wieder aufgehoben, was jedoch nicht immer vollständig möglich ist. Auch durch das Kühlen der Körpertemperatur und den Kontakt mit dem Fremdgewebe der Herz-Lungen-Maschine ist die Gerinnbarkeit Blutes nach einer herzchirurgischen Operation eingeschränkt. Insgesamt stellt sich also nach einem herzchirurgischen Eingriff das Problem sehr großer Wundflächen bei eingeschränkter Gerinnbarkeit des Blutes. Hierdurch sind die bei diesen Eingriffen auftretenden Nachblutungen, die bis zu 2000 ml in den ersten 24 Stunden nach der Operation betragen können, zu erklären. Durch den Ersatz des Blutverlustes mit Blutersatzpräparaten kann es zu einer weiteren Blutverdünnung und damit Blutungsneigung kommen.

Um dieses Blut, das sich vornehmlich im Perikardsack ansammelt, abzuleiten, werden die bereits erwähnten Drainagen eingelegt. Sind diese Drainagen verstopft oder kommen sie so zu liegen, daß nicht das gesamte Blut ablaufen kann, sammelt es sich im Perikard an. Bei weiterer Blutung wird, da das Perikard nicht dehnbar ist, durch die Blutung das Herz komprimiert. Durch diese Komprimierung kann sich das Herz nicht mehr richtig füllen und damit auch nicht mehr genug Blut durch den Körper pumpen. Daraus resultiert dann ein Blutdruckabfall, ein Anstieg des zentralen Venendrucks, ein Rückgang der Urinausscheidung und eine Kreislaufzentra- lisation. Im Röntgenbild kommt es zu einer Verbreiterung des Herzschattens. Diese Situation wird als Perikardtamponade bezeichnet. Sie ist für den Patienten akut lebensbedrohlich; erfolgt nicht sofort eine Reoperation mit Entlastung des Perikards durch Entfernen des angesammelten Blutes, so endet diese Komplikation tödlich.

Eine Perikardtamponade ist immer erst an ihrem klinischen Bild zu erkennen. Ein Röntgenbild des Brustkorbs mit Verbreiterung des Herzschattens zur Diagnostik ist allein nicht ausreichend. Die Durchführung einer Ultraschalluntersuchung ist nicht immer verläßlich, da nach einer Operation das zu untersuchende Gewebe oftmals schlecht dargestellt werden kann. Dazu kommt, daß sowohl die Röntgen- als auch die Ultraschalluntersuchung erst durchgeführt werden, wenn ein konkreter Verdacht besteht. Dieser Verdacht entsteht meist erst, wenn sich die obengenannten Symptome zeigen. Bis zum Verdacht und während der angeforderten Untersuchungen kann jedoch für den Patienten wertvolle Zeit verstreichen. Er kann sich währenddessen soweit verschlechtern, daß die Operation unter Notfallbedingungen vorgenommen werden muß. Eine Verzögerung der Operation oder gar die Notfall-Operation einer Perikardtamponade kann sich aufgrund von Schädigung anderer Organsysteme, wie Leber und Nieren, oder möglichen Infektionen für den Patienten erneut fatal auswirken und seine Liegedauer auf der Intensivstation deutlich verlängern.

Aufgabe der Erfindung ist es deshalb, eine Vorrichtung zur Verfügung zu stellen, mit der es möglich ist, den Patienten im Hinblick auf eine sich entwickelnde Perikardtamponade kontinuierlich zu überwachen und den Arzt oder das Pflegepersonal noch vor Ausbildung der klinischen Symptome zu alarmieren.

Diese Aufgabe wird gelöst durch eine Vorrichtung zur Feststellung eines Perikardergusses, bestehend aus einem Meßgerät, welches mit einer Drahtsonde zur Verankerung auf der rechten Herzkammer und mindestens einer weiteren Drahtsonde zur Verankerung in einem Bereich des Perikards verbunden ist, wobei das Meßgerät die Impedanz zwischen den Drahtsonden mißt und anzeigt und die gemessenen Impedanzwerte mit einem Null- bzw. Ausgangswert vergleicht und die Änderung der Impedanz anzeigt.

Impedanzmessungen zur Erfassung von Gewebeeigenschaften oder Ödemen sind in der Medizin bekannt. So beschreibt beispielsweise US 5,454,377 ein Verfahren zur Erfassung der Impedanz im Myokard. Dabei werden mittels zweier Elektroden, die in einem Teil des Myokards verankert werden, Strompulse erzeugt, die eine Spannung zwischen den Elektroden erzeugen. Durch eine ein Impedanzspektrum, welches charakteristisch für den Myokardzustand ist, umgerechnet werden.

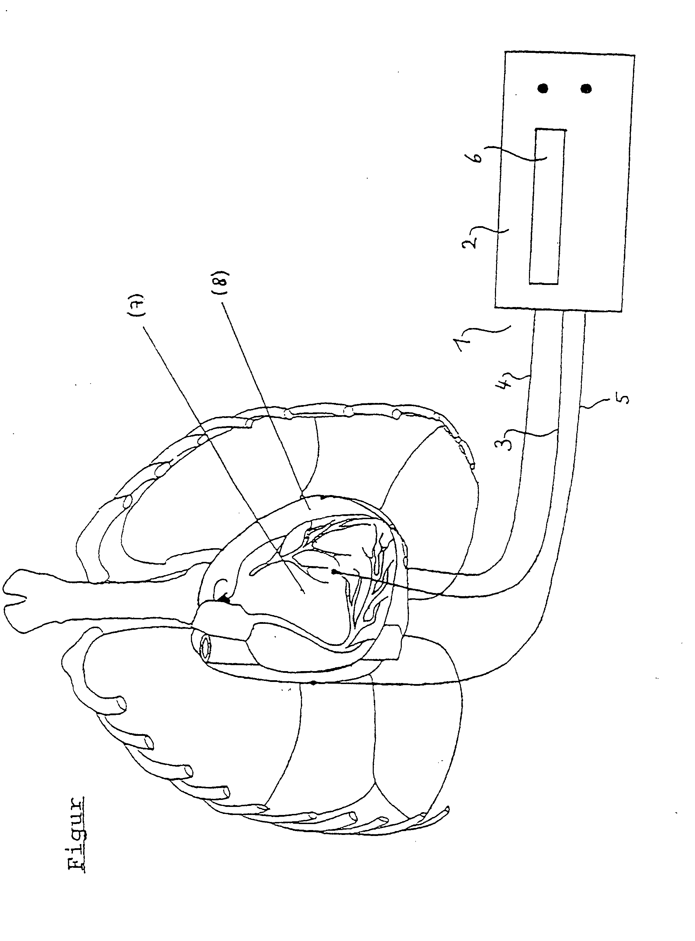

Die erfindungsgemäße Vorrichtung wird im folgenden anhand der Figur beschrieben.

Die Figur zeigt eine bevorzugte Ausführungsform der erfindungsgemäßen Vorrichtung zur Feststellung eines PerikardErgusses in Verbindung mit dem menschlichen Herzen.

Gemäß. Figur besteht die erfindungsgemäße Vorrichtung 1 aus einem Meßgerät 2, welches mit insgesamt drei Drahtsonden 3, 4 und 5 verbunden ist. Bei der ersten Drahtsonde 3 kann es sich um eine gewöhnliche passagere Herzschrittmachersonde handeln, die bei nahezu jeder herzchirugischen Operation vor Verschluß des Brustbeins auf den rechten Ventrikel 7 genäht und nach außen durch die Haut unterhalb des Brustbeins geführt wird. Gewöhnlich dient die Herzschrittmachersonde dazu, bei den häufig nach Herzoperationen eintretenden langsamen Rhythmusstörungen das Herz mit Hilfe dieser Sonde und einem externen Herzschrittmacher zu stimulieren und die gewünschte Herzfrequenz einstellen zu können. Die Drahtsonde 3 hat etwa 0,5 mm Durchmesser und ist zweipolig.

Die weiteren mit dem Meßgerät 2 verbunden Sonden 4 und 5 dienen der Verankerung im Perikardsack 8. Sie.können jeweils links und rechts vom Herzen in den perikardialen Sack eingenäht werden.

Nach Fixierung der Sonden 3 bis 5 in den entsprechenden Geweben ist es möglich, mit Hilfe der erfindungsgemäßen Vorrichtung durch eine Impedanzmessung zwischen den verschiedenen Sonden einen Erguß zu diagnostizieren. Kommt es bei der Verwendung der erfindungsgemäßen Vorrichtung zu einem Perikarderguß, so verändert das eingeflossene Blut oder Serum den Widerstand (bzw. Impedanz) zwischen den Sonden. Die Änderung der Impedanz zwischen den einzelnen Meßpunkten wird von dem Meßgerät 2 gemessen und in einer Anzeige 6 angezeigt. Da sowohl zwischen Ventrikel- und den Perikardsonden, als auch zwischen den Perikardsonden direkt gemessen werden kann, können sowohl Ausdehnung als auch Lage des Ergusses festgestellt werden. Voraussetzung hierzu ist ein Null- bzw. Ausgangswert, bei dem kein Erguß vorhanden ist. Dieser Ausgangswert kann problemlos vor Verschluß des Brustkorbs gemessen werden, da hierbei nochmals mit Hilfe eines Saugers das Perikard leergesaugt wird. Gegen den unmittelbar danach gemessenen Wert wird nun jederzeit von dem Meßgerät 2 verglichen. Überschreitet die Differenz einen gewissen Schwellwert, gibt das Meßgerät 2 eine Warnung, wird ein weiterer Schwellwert überschritten, erfolgt Alarm.

Die jeweiligen Schwellwerte müssen entsprechend erprobt und im Versuch ermittelt werden. Sind diese an einem entsprechend großen Patientenkollektiv erhoben, kann die Vorrichtung an jedem Patienten ohne langwierige Kalibration eingesetzt werden. Es ist, wie bereits erwähnt, eine einmalige intraoperative Nullpunktmessung notwendig.

Die notwendigen Sonden 3, 4 und 5 können, genau wie eine Schrittmachersonde, nach dem Ende der Anwendung durch die Haut gezogen und entfernt werden.

Die Vorrichtung 1 muß sicher gegenüber Hochfrequenzstrom (elektrisches Messer) und auch während Schrittmacherbetrieb einsetzbar sein; zudem darf keine Beeinflussung des Schrittmachers auftreten. Weiter sollte sie möglichst klein und leicht sein, damit der Patient damit auch mobilisierbar bleibt. Durch das leichte Gewicht der elektronischen Bauteile des Impedanzmeßgerätes 2 und der verwendeten Sonden 3, 4, 5 ist die erfindungsgemäße Vorrichtung in handlicher Größe und mit geringem Gewicht als von einem Arzt oder dem Patienten leicht mitzuführende Vorrichtung herstellbar.

Da die Vorrichtung als Routinegerät konzipiert ist, können vor allem die Verbrauchsmaterialien möglichst günstige Einmalprodukte sein, wie es beispielsweise bei den bisher verwendeten aufnähbaren Schrittmachersonden der Fall ist.

Die erfindungsgemäße Vorrichtung bietet für den postoperativen herzchirugischen Patienten ein mehr an Sicherheit, da die Ausbildung eines Perikardergusses mit gefährlicher Tamponade kontinuierlich überwacht und unmittelbar festgestellt werden kann. Die Größe eines Ergusses ist direkt meßbar. Hat der Erguß eine bestimmte Größe erreicht, wird Alarm ausgelöst und eine weiterführende Diagnostik mittels Echokardiographie oder anderer Methoden kann ohne weitere Verzögerung sofort eingeleitet werden. Das Einbringen der Sonden ist unkompliziert und von einem routinierten Herzchirurgen einfach zu bewerkstelligen.

## Patentansprüche

1. Vorrichtung zur Feststellung eines Perikardergusses, bestehend aus einem Meßgerät (2), welches mit einer Drahtsonde zur Verankerung auf der rechten Herzkammer und mindestens einer weiteren Drahtsonde (4) zur Verankerung in einem Bereich des Perikards verbunden ist, wobei das Meßgerät (2) die Impedanz zwischen den Drahtsonden mißt und anzeigt und die gemessenen Impedanzwerte mit einem Null- bzw. Ausgangswert vergleicht und die Änderung der Impedanz anzeigt.

2. Vorrichtung nach Anspruch 1, **dadurch gekennzeichnet, daß** insgesamt zwei weitere Drahtsonden (4, 5) zur Verankerung in verschiedenen Bereichen des Perikards vorgesehen sind.

3. Vorrichtung nach einem der vorherigen Ansprüche, **dadurch gekennzeichnet, daß** das Meßgerät (2) bei Überschreitung eines bestimmten Schwellwertes der Impedanzänderung ein Tonsignal abgibt.

4. Vorrichtung nach einem der vorherigen Ansprüche, **dadurch gekennzeichnet, daß** sie als tragbares Gerät konzipiert ist.

5. Vorrichtung nach einem der vorhergehenden Ansprüche, **dadurch gekennzeichnet, daß** die Drahtsonde (3) zur Verankerung auf der rechten Herzkammer eine gewöhnliche passagere Herzschrittmachersonde ist, die auf den rechten Ventrikel (7) genäht werden kann.

## Claims

1. Device for detecting a pericardial effusion, comprising a measuring instrument (2), which is connected to a wire probe for anchorage to the right ventricle and at least one further wire probe (4) for anchorage in a region of the pericardium, wherein the measuring instrument (2) measures and displays the impedance between the wire probes and compares the measured impedance values with a zero or starting value and displays the change in impedance.

2. Device according to Claim 1, **characterised in that** two further wire probes (4, 5) are provided overall for anchorage in different regions of the pericardium.

3. Device according to one of the preceding claims, **characterised in that** the measuring instrument (2) emits an audio signal when a specific threshold value of the change in impedance has been exceeded.

4. Device according to one of the preceding claims, **characterised in that** it is designed as a portable device.

5. Device according to one of the preceding claims, **characterised in that** the wire probe (3) for anchorage to the right ventricle of the heart is a standard short-term pacemaker probe, which can be sewn onto the right ventricle (7).

## Revendications

1. Dispositif de constatation d'un épanchement péricardique, composé d'un appareil de mesure (2) qui est relié à une sonde à transmission filaire pour un ancrage sur le ventricule cardiaque de droite et au moins à une autre sonde à transmission filaire (4) pour un ancrage dans une région du péricarde, dans lequel l'appareil de mesure (2) mesure et indique l'impédance entre les sondes à transmission filaire et compare les valeurs de l'impédance mesurée à une valeur nulle, respectivement une valeur de départ, et indique la variation d'impédance.

2. Dispositif selon la revendication 1, **caractérisé en ce que** deux autres sondes à transmission filaire (4, 5) sont prévues pour un ancrage dans différentes régions du péricarde.

3. Dispositif selon l'une des revendications précédentes, **caractérisé en ce que** l'appareil de mesure (2) génère un signal sonore en cas de dépassement d'une certaine valeur de seuil de la variation d'impédance.

4. Dispositif selon l'une des revendications précédentes, **caractérisé en ce qu'**il est conçu comme un appareil portable.

5. Dispositif selon l'une des revendications précédentes, **caractérisé en ce que** la sonde à transmission filaire (3) pour ancrage sur le ventricule cardiaque de droite est une sonde de passage habituel dans le stimulateur cardiaque qui peut être cousue sur le ventricule de droite (7).
